# EUROPEAN PATENT APPLICATION

(11) **EP 3 888 698 A1**
(43) Date of publication of application: **06.10.2021**
(21) Application number: 21167054.2
(22) Date of filing: 06.04.2021
(51) Int. Cl.: A61L 2/10, A41D 13/11, A61L 2/28

(54) **FACE MASK SYSTEM WITH CONDITION INDICATOR**

(30) Priority: 02.04.2020 US 202063004140 P
(71) Applicant: ReadyDock, Inc., Avon, CT 06001 (US)
(72) Inventor: Kersey, Alan, Glastonbury, 06073 (US); Engelhardt, David, Canton, 06073 (US)
(74) Representative: Dehns

(57) **Abstract**

A face mask system (44) is provided that includes a face mask (12) and an indicator (42). The indicator (42) is arranged with the face mask (12). The indicator (42) may be configured to indicate the face mask (12) is no longer recommended for use following a first level of exposure of the face mask (12) to ultraviolet light. In addition, or alternatively, the indicator (42) may be configured to change its appearance when a first number of treatment cycles have been performed on the face mask (12) using the ultraviolet light. In addition, or alternatively still, the indicator (42) may be configured to visually indicate a mechanical property of the face mask (12) is preserved following another level of exposure of the face mask (12) to the ultraviolet light.

## Description

### BACKGROUND OF THE DISCLOSURE

### 1. Technical Field

This disclosure relates generally to face masks and, more particularly, to disinfection and/or sanitization of face masks.

### 2. Background Information

Ultraviolet light typically refers to light at wavelengths in a band of about 10nm to about 400nm. There are several sub-bands within the ultraviolet light band. These ultraviolet light sub-bands include a UVA light sub-band ("UVA light"), a UVB light sub-band ("UVB light") and a UVC light sub-band ("UVC light"). The UVC light typically refers to light at wavelengths in a band of about 100nm to about 280nm. Some UVC light wavelengths are known to produce germicidal effects on certain harmful microorganisms (e.g., bacterial viruses, mold, and other pathogens) when those microorganisms are exposed to the UVC light for a sufficient period of time at a sufficient intensity. UVC light may therefore be used to disinfect food and water products. The UVC light, for example, may be absorbed by one or more constituents (e.g., proteins, DNA molecules or the like) within the microorganisms on the food or in the water products. This UVC light absorption may cause the constituents to morph into a form where the ability of the microorganisms to replicate or perform other vital biological functions is negatively affected. The UVC light may also or alternatively destroy one or more of the microorganisms.

Face masks are used by healthcare professionals as well as other individuals to protect those individuals from potentially harmful airborne microorganisms as well as other particulates such as dust. A face mask may be worn by an individual, for example, to filter air inhaled and exhaled by the individual. This filtering may protect the individual wearing the face mask from airborne microorganisms and other particulates in the surrounding air. The filtering may also protect other people near the individual from microorganisms carried by the individual.

A typical face mask is designed for a single use in order to reduce potential for spreading harmful microorganisms which may accumulate on surfaces of the face mask. A healthcare professional may therefore go through many face masks during a single day where, for example, that professional wears a new face mask for interacting with each new patient. Thus, where a healthcare professional interacts with many patients during a day, the cost for providing the faces mask may be relatively high. In addition, during an emergency such as the COVID-19 pandemic, medical supplies such as face masks may become scarce. There is a need in the art therefore for systems and method to provide for reusable face masks.

### SUMMARY OF THE DISCLOSURE

According to an aspect of the present disclosure, a face mask system is provided that includes a face mask and an indicator. The indicator is arranged with the face mask. The indicator is configured to indicate the face mask is no longer recommended for use following a first level of exposure of the face mask to ultraviolet light.

According to another aspect of the present disclosure, another face mask system is provided that includes a face mask and an indicator. The indicator is arranged with the face mask and has an appearance. The indicator is configured to change the appearance when a first number of treatment cycles have been performed on the face mask using ultraviolet light. The first number of treatment cycles may be greater than one.

According to still another aspect of the present disclosure, another face mask system is provided that includes a face mask and an indicator. The indicator is arranged with the face mask. The indicator is configured to visually indicate a mechanical property of the face mask is preserved following a first level of exposure of the face mask to ultraviolet light.

In at least some embodiments, the indicator may also be configured to indicate the mechanical property of the face mask could be (or is) degraded following a second level of exposure of the face mask to the ultraviolet light. The second level of exposure of the face mask to the ultraviolet light may be greater than the first level of exposure of the face mask to the ultraviolet light.

In at least some embodiments, the indicator may also be configured to maintain the appearance when a second number of treatment cycles have been performed on the face mask using the ultraviolet light. The second number of treatment cycles may be less than the first number of treatment cycles.

In at least some embodiments, the change in appearance may indicate the face mask is no longer recommended for use.

In at least some embodiments, the change in appearance may be a change in color from a first color to a second color. The first color may be green and/or the second color may be red.

In at least some embodiments, the change in appearance may correspond with an increased likelihood that a mechanical property of the face mask has been degraded from exposure to the ultraviolet light.

In at least some embodiments, the first level of exposure of the face mask to the ultraviolet light may correspond to a first number of disinfection cycles performed on the face mask with the ultraviolet light.

In at least some embodiments, the first number of disinfection cycles may be greater than one, two, three or otherwise.

In at least some embodiments, the face mask may no longer be recommended for use when a mechanical property of the face mask could be (or is) degraded following the first level of exposure to the ultraviolet light.

In at least some embodiments, the mechanical property may be or otherwise include fit of the face mask.

In at least some embodiments, the mechanical property may be or otherwise include filtering capability of the face mask.

In at least some embodiments, the indicator may also be configured to indicate the face mask is acceptable for use following a second level of exposure of the face mask to the ultraviolet light. The second level of exposure of the face mask to the ultraviolet light may be less than the first level of exposure of the face mask to the ultraviolet light.

In at least some embodiments, the ultraviolet light may be UVC light.

In at least some embodiments, the indicator may be configured to visually indicate the face mask is no longer recommended for use following the first level of exposure of the face mask to the ultraviolet light.

In at least some embodiments, the indicator may be configured to change colors following the first level of exposure of the face mask to the ultraviolet light. For example, the indicator may change from a first color to a second color following the first level of exposure of the face mask to the ultraviolet light. The first color may be green and/or the second color may be red.

In at least some embodiments, the indicator may be attached to an exterior of the face mask.

In at least some embodiments, the indicator may be integrated with the face mask.

In at least some embodiments, the indicator may be configured from or otherwise include photochromic material.

In at least some embodiments, the face mask may be configured as an N95 face mask.

The present disclosure may include any one or more of the individual features and embodiments disclosed above and/or below, alone or in any combination thereof.

The foregoing features and the operation of the invention will become more apparent in light of the following description and the accompanying drawings.

### BRIEF DESCRIPTION OF THE DRAWINGS

FIG. 1 is a schematic illustration of a system for treating a face mask.
FIG. 2 is a partial schematic illustration of the face mask supported within a treatment area of the face mask treatment system.
FIG. 3 is a partial perspective illustration of the face mask treatment system.
FIG. 4 is a flow diagram of a method for treating a face mask.
FIG. 5 is an illustration of the face mask configured with a face mask condition indicator.
FIGS. 6A and 6B schematically illustrate a change in appearance (e.g., color and/or pattern) of the face mask condition indicator following exposure to a certain level of ultraviolet light.
FIG. 7 is a flow diagram of a method for using and treating the face mask.
FIGS. 8A-8C schematically illustrate various configurations with which the face mask condition indicator may be arranged with the face mask.

### DETAILED DESCRIPTION

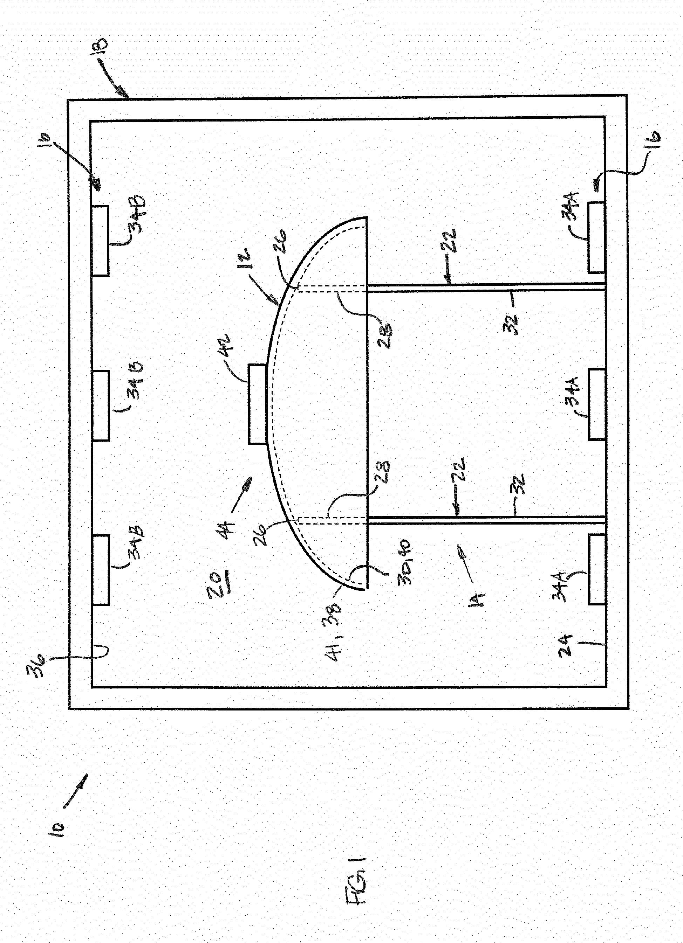

FIG. 1 illustrates a system 10 for treating a face mask 12 using ultraviolet (UV) light; e.g., UVC light. This ultraviolet light treatment is operable to at least partially or completely disinfect and/or sanitize the face mask 12. The ultraviolet light treatment may thereby facilitate extended use (e.g., re-use) of the face mask 12. The face mask 12, for example, may be used for a certain number of intervals before discarding the face mask 12, where the face mask 12 is treated using the treatment system 10 following each interval. Each interval may correspond to a certain period of time (duration) such as, but not limited to, a certain number of minutes, a certain number of hours or a certain number of days. Each interval may alternatively correspond to a certain use such as, but not limited to, one or more interactions with an individual (e.g., a patient, a sick or injured person), presence within a room or a ward, or performance of a task (e.g., a medical treatment, administration of one or more medical tests).

The treatment system 10 of FIG. 1 includes a support structure 14 and an ultraviolet (e.g., UVC) light treatment device 16. The treatment system 10 of FIG. 1 also includes a base structure 18 (e.g., a housing, an enclosure, a stage, etc.) with a treatment area 20, which treatment area 20 may be an open or enclosed volume within the base structure 18.

The support structure 14 is configured to support the face mask 12 (or multiple face masks) within the treatment area 20 during operation of the treatment system 10. The support structure 14 of FIG. 1, for example, is a frame that includes one or more face mask positioning elements 22; e.g., pins, pedestals, stands, etc. These positioning elements 22 are attached to a floor 24 of base structure 18 and its treatment area 20. Each of the positioning elements 22 projects vertically out from the treatment area floor 24 to a respective distal end 26.

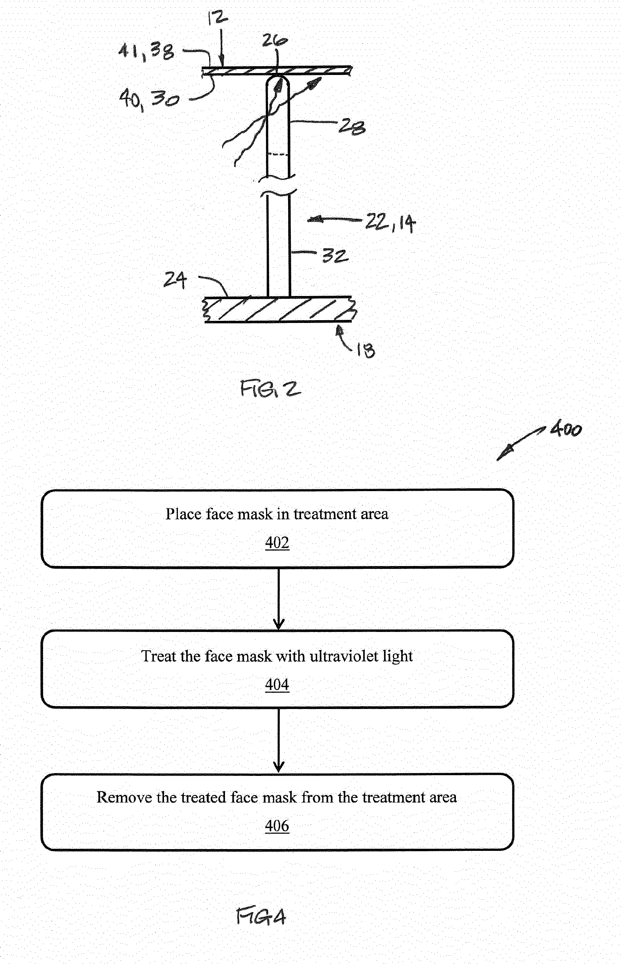

Referring to FIG. 2, each of the positioning elements 22 may be configured with at least an ultraviolet light transparent tip 28 at the distal end 26. This transparent tip 28 is configured to facilitate transmission of the ultraviolet (e.g., UVC) light therethrough to prevent or reduce shadowing of the ultraviolet light on a portion of a bottom (e.g., interior) surface 30 of the face mask 12 that engages (e.g., contacts) and/or is near (e.g., adjacent and/or surrounds) the respective positioning element 22. At least the transparent tip 28, for example, may be constructed from ultraviolet transparent material; e.g., UVC transparent material. Of course, in other embodiments, an entirety of each positioning element 22 may be constructed from the ultraviolet transparent material.

Each positioning element 22 may also be flexible and resilient or deformable (e.g., manipulatable). At least a base portion 32 of each positioning element 22, for example, may be constructed from flexible material; e.g., flexible polymer material. Thus, when an operator places the face mask 12 (or face masks) within the treatment area 20, one or more the positioning elements 22 may resiliently slightly give / deflect when coming into contact with the operator's hand. This may facilitate easier face mask placement as well as reduce potential for injury to the operator's hand. Where the positioning element 22 is deformable, the flexibility of the positioning element 22 may also facilitate accommodation of the support structure 14 to different face mask designs. The present disclosure, however, is not limited to such exemplary flexible configurations. For example, one or more of the positioning elements 22 may alternatively be rigid.

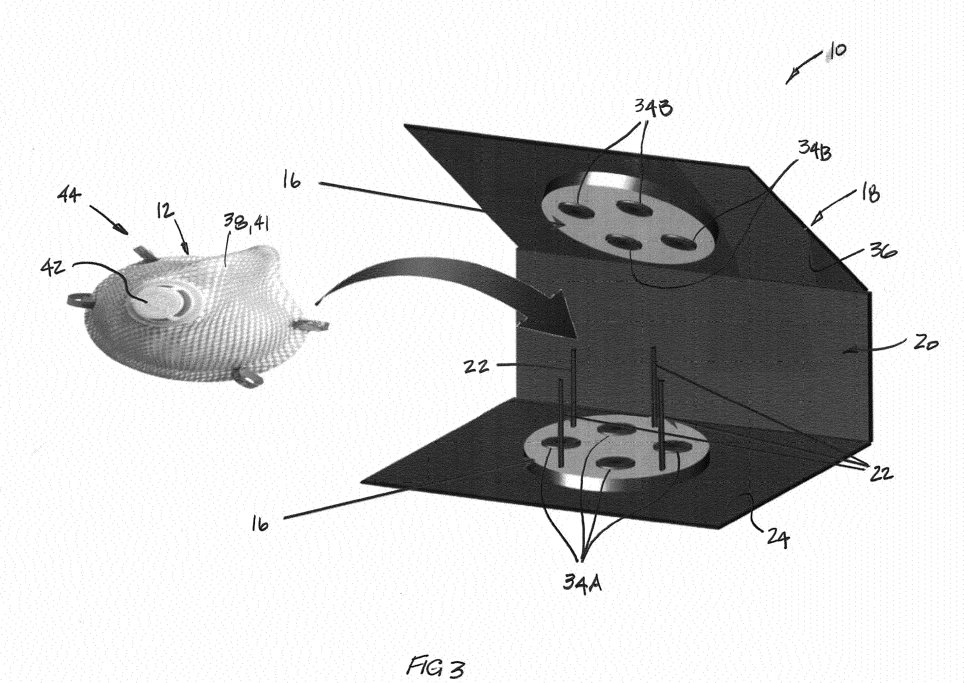

Referring to FIG. 3, the treatment device 16 is configured to apply the ultraviolet light treatment (e.g., UVC light treatment) to the face mask 12 (or face masks) within the treatment area 20. The treatment device 16 of FIG. 3, for example, includes one or more ultraviolet light sources 34A and 34B (generally referred to as "34"); e.g., UVC light sources. Each light source 34 is configured to generate, emit and/or otherwise provide the ultraviolet (e.g., UVC) light for the treatment of the face mask 12 (or face masks). Each light source 34, for example, may be or include an ultraviolet light emitting diode; e.g., a UVC-LED. Such a UVC-LED may be configured to produce wavelengths of light within the UVC light sub-band; e.g., between about or exactly 100nm and about or exactly 280nm. UVC-LEDs that produce wavelengths of light between about or exactly 222nm and about or exactly 254nm may be particularly useful for face mask disinfection and/or sanitization.

One or more of the light sources (e.g., 34A) may be arranged to be below the face mask 12 (or face masks) within the treatment area 20. The lower light sources 34A of FIG. 3, for example, are located at (e.g., on, adjacent or proximate) the treatment area floor 24. These lower light sources 34A are configured to direct the ultraviolet (e.g., UVC) light in a general upward direction. One or more of the light sources (e.g., 34B) may also or alternatively be arranged to be above the face mask 12 (or face masks) within the treatment area 20. The upper light sources 34B of FIG. 3, for example, are located at (e.g., on, adjacent or proximate) a ceiling 36 (e.g., a top and/or lid) of the base structure 18 and its treatment area 20. These upper light sources 34B are configured to direct the ultraviolet (e.g., UVC) light in a general downward direction. With such an arrangement, the treatment device 16 may be operable to (e.g., concurrently) treat both a face mask top (e.g., exterior) surface 38 and the face mask bottom (e.g., interior) surface 30; see FIG. 1.

FIG. 4 is a flow diagram of a method 400 for treating the face mask 12 using a system such as, but not limited to, the treatment system 10. For ease of description, the method 400 is described for treatment of a single face mask. However, the method 400 could also be used for treating multiple face masks 12 concurrently where the multiple face masks are arranged within the treatment area 20.

In step 402, a previously used or otherwise contaminated face mask 12 is placed within the treatment area 20. The operator, for example, may dispose the face mask 12 on the support structure 14 where the tips 28 of the positioning elements 22 engage the bottom (e.g., interior) surface 30 of the face mask 12; e.g., see FIGS. 1 and 2. While FIG. 1 depicts the face mask bottom surface 30 as being an interior (inner) surface 40 of the face mask 12, the present disclosure is not limited to such an exemplary arrangement. For example, in other embodiments, the face mask 12 may be rearranged such that its bottom surface that engages the positioning element tips is an exterior (outer) surface 41 of the face mask 12.

In step 404, the face mask 12 is treated with the ultraviolet light; e.g., UVC light. The light sources 34, for example, are activated (e.g., turned on) such that the ultraviolet (e.g., UVC) light is projected against each surface (e.g., 40, 41) of the face mask 12. After exposure of the face mask 12 to a certain intensity of the ultraviolet (e.g., UVC) light for a certain period of time (duration), the face mask 12 may be at least partially (e.g., substantially) or completely disinfected and/or sanitized. During treatment, for example, the ultraviolet (e.g., UVC) light may be absorbed by one or more constituents (e.g., proteins, DNA molecules, or the like) within one or more microorganisms (e.g., bacterial viruses, mold, and other pathogens) on the face mask 12. This absorption may cause one or more of the constituents to morph into a form where the ability of the respective microorganism to replicate and/or perform other vital biological functions is negatively affected. The absorption may also or alternatively destroy one or more of the microorganisms on the face mask 12.

In step 406, the treated face mask 12 is removed from the treatment area 20. This treated face mask 12 may now be used / reused by an individual (e.g., a healthcare professional, etc.) while still providing proper protection; e.g., protection that satisfies a standard level associated with that type of face mask 12. In this manner, a single face mask may be treated and then reused for multiple cycles / intervals of use. This may extend a useful service life of the face mask 12 as well as reduce demand for face masks in a stressed environment such as during a pandemic and/or where face masks may be in high demand but in short supply.

While the ultraviolet (e.g., UVC) light can be used for treating the face mask 12, the ultraviolet (e.g., UVC) light can also begin to degrade / deteriorate one or more mechanical properties (e.g., qualities) of the face mask 12 following extended over exposure. For example, extended over exposure to the ultraviolet (e.g., UVC) light may degrade / deteriorate material of the face mask 12 such that face mask fitment is compromised; e.g., the face mask 12 may no longer fit properly on / against an individual's face. Extended over exposure to the ultraviolet (e.g., UVC) light may also or alternatively degrade / deteriorate the face mask material such that face mask filtering capability / performance is compromised; e.g., the face mask 12 may no longer be capable of filtering air passing therethrough according to an associated standard for that face mask 12. Individuals may therefore be hesitant to use / reuse a face mask that has been treated with ultraviolet light; e.g., UVC light.

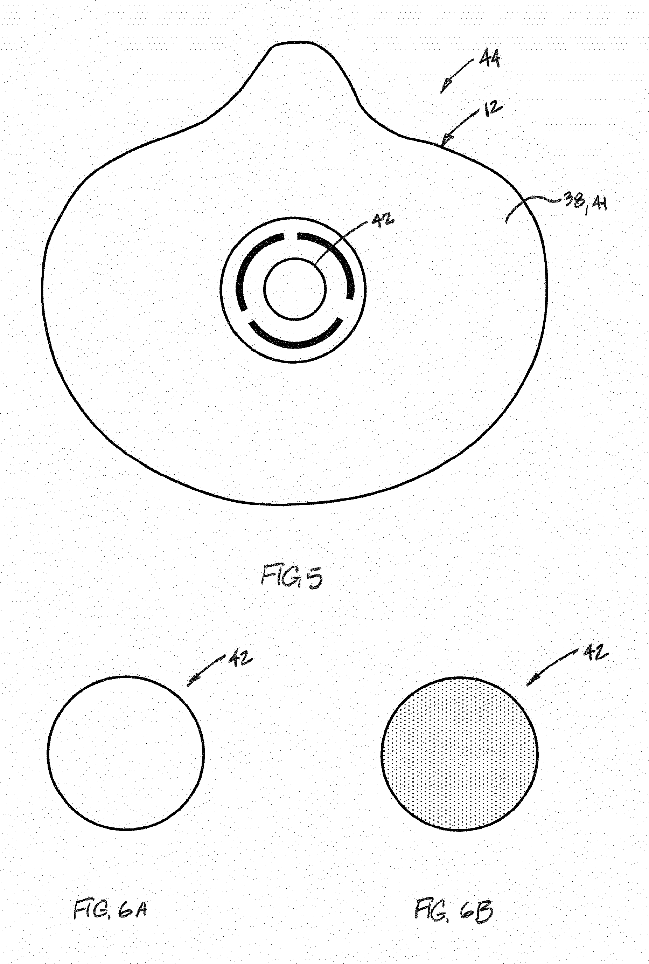

To ensure safe face mask usage and/or to alleviate hesitancy associated with using an ultraviolet (e.g., UVC) light treated face mask, the face mask 12 of FIG. 5 is provided with a face mask condition indicator 42 such as, for example, a face mask condition identification marker. More particularly, FIG. 5 illustrates a face mask system 44 which include the face mask 12 and the indicator 42. This indicator 42 is configured to indicate when the face mask 12 may be acceptable for use and/or when the face mask 12 may no longer recommended for use. For example, it may be known or predicted that the material of the face mask 12 may begin to degrade / deteriorate when the face mask 12 has a certain threshold level of exposure to the ultraviolet (e.g., UVC) light; e.g., exposure to the ultraviolet (e.g., UVC) light at a certain intensity for a certain period of time, including multiple independent exposures that create a collective exposure. This threshold level of ultraviolet (e.g., UVC) light exposure may correspond to when (or about when with provision of a safety factor) one or more mechanical properties of the face mask 12, such as those listed above or otherwise, begin to degrade / deteriorate. Therefore, where the face mask 12 is exposed to an acceptable level of exposure to the ultraviolet (e.g., UVC) light (e.g., a level that is less than the threshold level), the indicator 42 may indicate the face mask 12 is still acceptable for use. However, where the face mask 12 is exposed to an unacceptable level of exposure to the ultraviolet (e.g., UVC) light (e.g., a level that is equal to or greater than the threshold level), the indicator 42 may indicate the face mask 12 is no longer recommended (or suitable) for use.

The indicator 42 is configured to (e.g., passively) measure / detect a total (e.g., accumulative) level of exposure of the face mask 12 to the ultraviolet light. The indicator 42, for example, may be configured to measure / detect the total level of exposure of the face mask 12 to ultraviolet light in the UVC light sub-band (e.g., between about or exactly 100nm and about or exactly 280nm), or in a select portion of the UVC light sub-band (e.g., between about or exactly 222nm and about or exactly 254nm). The indicator 42 is also configured to provide an indication based on the measured level of exposure to the ultraviolet (e.g., UVC) light.

The indicator 42 may be constructed from or otherwise include ultraviolet (e.g., UVC) light sensitive (e.g., reactive) material. An example of such light sensitive material is, but is not limited to, photochromic material; e.g., a photochromic ink, a photochromic polymer, a photochromic color indicator, etc. This light sensitive material may be tailored / selected such that an appearance of the light sensitive material (e.g., substantially only) changes when exposed to a total level of ultraviolet (e.g., UVC) light that is equal to or greater than the threshold level of ultraviolet (e.g., UVC) light exposure; e.g., see change of indicator appearance from FIG. 6A to FIG. 6B. This change in the appearance of the indicator 42 may be a change in a color (e.g., from green to red) of the light sensitive material (schematically depicted by FIGS. 6A and 6B). The change in the appearance of the indicator 42 may also or alternatively be a change in a pattern presented (e.g., displayed) by the light sensitive material (schematically depicted by FIGS. 6A and 6B); e.g., a hidden pattern may appear. This pattern may be an arrangement of lines and/or dots, a picture, text, etc. The light sensitive material may also be tailored / selected such that the appearance (e.g., color, pattern, etc.) of the light sensitive material is maintained (stays the same) when exposed to a total level of ultraviolet (e.g., UVC) light that is less than the threshold level of ultraviolet (e.g., UVC) light exposure. With such a configuration, the indicator 42 is operable to visually indicate when the face mask 12 is acceptable for use and/or when the face mask 12 is no longer recommended for use. In other words, the indicator 42 can convey the current condition of the face mask. In a particularly useful embodiment, the indicator 42 may have a "binary" response when exposed to a total level of ultraviolet (e.g., UVC) light that is equal to or greater than the threshold level of ultraviolet (e.g., UVC) light exposure. For example, upon the threshold being met the indicator displays information (e.g., the word "DISPOSE") or a symbol (e.g., an image of a mask with an "X" through it) not present before the threshold is met, or prior to the threshold is met a mask is shown, and after the threshold is met a red "X" is shown over the mask, etc. These are examples of binary responses, and the present disclosure is not limited thereto. Such a binary response may facilitate the user readily understanding when the mask is acceptable and when it should be discarded, as well as people in proximity to the mask wearer.

Depending upon the specific intensity and/or duration of ultraviolet (e.g., UVC) light exposure during face mask treatment, the threshold level of ultraviolet (e.g., UVC) light exposure may correspond to a total level of ultraviolet (e.g., UVC) light exposure accumulated over a number of treatment cycles (e.g., disinfection and/or sanitization cycles), where the number of treatment cycles may be one (1), two (2), three (3), four (4), five (5) or more. Each treatment cycle (e.g., each performance of the method 400) performed on the face mask 12, for example, may subject that face mask to N times the threshold level of ultraviolet (e.g., UVC) light exposure, where N is a fraction. For example, where N is one-half (1/2), the face mask 12 may undergo two (2) treatment cycles before reaching the threshold level of ultraviolet (e.g., UVC) light exposure. Thus, the face mask 12 may be acceptable for reuse following the first treatment cycle; but may not be recommended for reuse following the second treatment cycle. In another example, where N is one-third (1/3), the face mask 12 may undergo three (3) treatment cycles before reaching the threshold level of ultraviolet (e.g., UVC) light exposure. Thus, the face mask 12 may be acceptable for reuse following a first treatment cycle and a second treatment cycle; but may not be recommended for reuse following the third treatment cycle. More generally, where N is one-over-X (1/X), the face mask 12 may undergo X number of treatment cycles before reaching the threshold level of ultraviolet (e.g., UVC) light exposure. Thus, the face mask 12 may be acceptable for reuse following X-minus-one (X-1) treatment cycles; but may not be recommended for reuse following the X^{th} treatment cycle. Generally, X may be equal to one (1), or alternatively any whole number greater than one; e.g., 2, 3, 4, 5, 6, 7, 8, 9, 10 or so on up to an upper bound.

With the foregoing in mind, the indicator 42 may provide its visual indication of whether or not the face mask 12 is acceptable for further use or should be discarded based on a number of treatment cycles (e.g., disinfection and/or sanitization cycles) to which that face mask 12 has been subjected. The indicator 42, for example, may be configured to visually indicate (e.g., via color such as green, pattern or otherwise) the face mask 12 may be acceptable for continued use where a total level of ultraviolet (e.g., UVC) light exposure to the face mask 12 accumulated over a first number of treatment cycles is less than the threshold level of ultraviolet (e.g., UVC) light exposure. The indicator 42 may also or alternatively be configured to visually indicate (e.g., via color such as red, pattern or otherwise) the face mask 12 may no longer be recommended for use / should be discarded where a total level of ultraviolet (e.g., UVC) light exposure to the face mask 12 accumulated over a second number of treatment cycles is equal to or greater than the threshold level of ultraviolet (e.g., UVC) light exposure, where the second number is greater than the first number. Thus, the indicator 42 may be configured as a treatment cycle counter for the face mask 12.

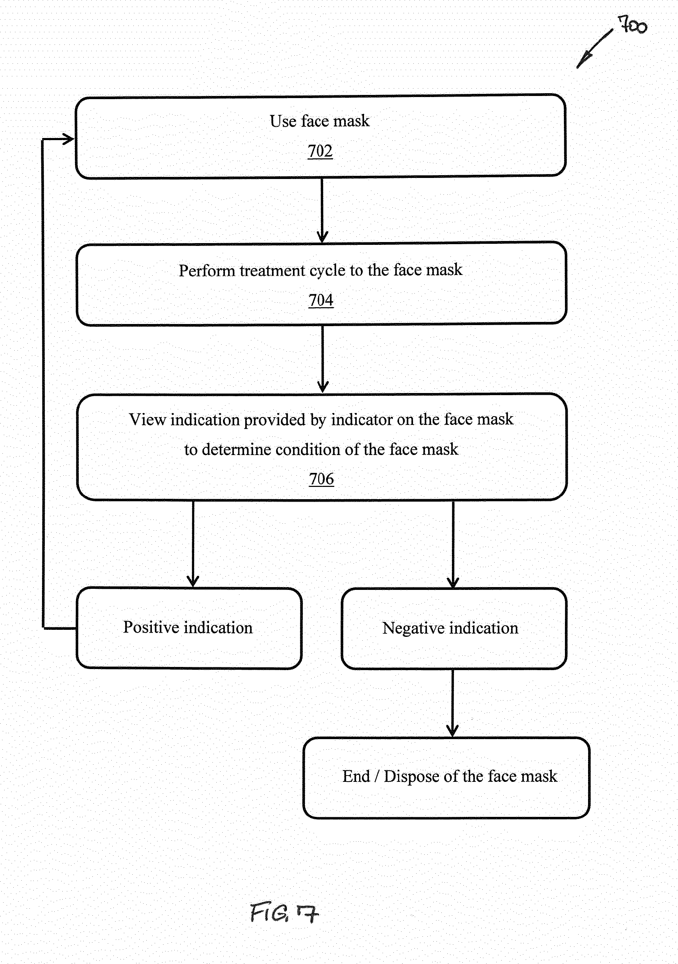

FIG. 7 is a flow diagram of a method 700 involving a face mask system such as the face mask system 44. In step 702, an individual uses the face mask 12 for a task. In step 704, following performance of the task in the step 702, the individual (or another individual) facilitates the method 400 in order to treat (e.g., disinfect and/or sanitize) the face mask 12. In step 706, following the performance of the treatment cycle in the step 704, the individual (or another individual) views the indication provided by the indicator 42 to determine the condition of the treated face mask 12. Where the indicator 42 indicates the face mask 12 may still be acceptable for further use - a positive indication, the individual (or another individual) may reuse the face mask 12. Thus, steps 702, 704 and 706 may be repeated. However, where the indicator 42 indicates the face mask 12 may no longer be recommended for further use - a negative indication (e.g., may no longer be suitable for proper fitment and/or filtering), the individual (or another individual) may discard (throw away, recycle) or otherwise dispose of the face mask 12. Thus, an individual who picks up the face mask system 44 of the present disclosure will be able to tell whether the face mask system 44 and its face mask 12 are suitable for use whether or not that individual knows the previous treatment history of the face mask system 44 / the face mask 12.

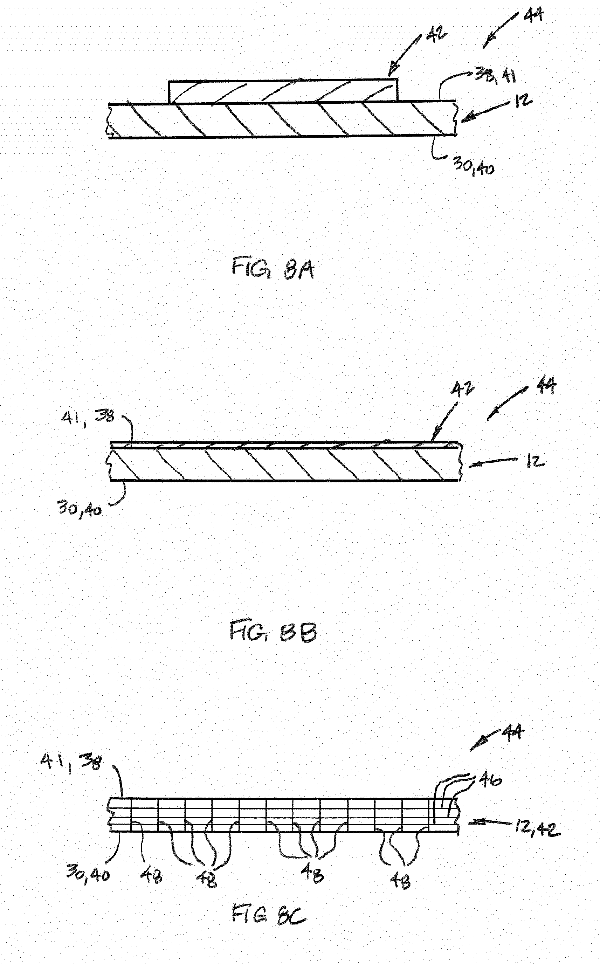

In some embodiments, referring to FIG. 8A, the indicator 42 may be attached to the face mask 12; e.g., a body of the face mask 12. The indicator 42, for example, may be formed as a discrete element (e.g., a sticker, a patch, a badge, etc.) that is subsequently bonded (e.g., adhered) or otherwise attached to the face mask material at, for example, the face mask exterior surface 38.

In some embodiment, referring to FIG. 8B, the indicator 42 may be applied onto the face mask 12. The indicator 42, for example, may be configured as a coating (e.g., paint, ink, etc.) that is applied (e.g., painted, printed, sprayed, etc.) onto the face mask material at, for example, the face mask exterior surface 38. With such embodiments, the indicator 42 may be provided in addition to (e.g., discrete from) other labeling and/or marking on the face mask 12 or as part of the labeling and/or marking. Such labeling and/or marking may be used to identify a manufacturer of the face mask 12, the type of face mask or various other information.

In some embodiments, referring to FIG. 8C, the indicator 42 may be integrated with the face mask material (schematically shown). For example, one or more elements 46 (e.g., filaments, strands, particles, etc.) of photochromic material may be arranged (e.g., interwoven, intermixed, etc.) with one or more elements 48 (e.g., filaments, strands, particles, etc.) of other (e.g., typical, traditional) face mask material to form the face mask body. In another example, each element 48 of the face mask material be formed from or otherwise include (e.g., be coated with) the photochromic material. The present disclosure, of course, is not limited to the foregoing exemplary integral indicator configurations.

The face mask 12 may be configured as any type of face mask / face covering that filters air inhaled by a user and/or exhaled by the user during breathing. Examples of the face mask 12 include, but are not limited to, a respirator face mask (e.g., an N95 face mask or a KN95 face mask), a cone-style face mask, a dust and/or debris filtering face mask, a surgical face mask and a cloth face mask.

Various types of ultraviolet (e.g., UVC) light sensitive materials such as photochromic materials are known in the art which may be selected and/or tailored to provide a visual indication as described above. Examples of such light sensitive material are disclosed in U.S. Patent No. 7,708,396 and U.S. Patent No. 7,247,262. The present disclosure, however, is not limited to such exemplary light sensitive materials nor to indicators made from such light sensitive materials.

While various embodiments of the present disclosure have been described, it will be apparent to those of ordinary skill in the art that many more embodiments and implementations are possible within the scope of the disclosure. For example, the present disclosure as described herein includes several aspects and embodiments that include particular features. Although these features may be described individually, it is within the scope of the present disclosure that some or all of these features may be combined with any one of the aspects and remain within the scope of the disclosure. Accordingly, the present disclosure is not to be restricted except in light of the attached claims and their equivalents.

## Claims

1. A face mask system, comprising:
a face mask; and
an indicator arranged with the face mask, the indicator configured to indicate the face mask is no longer recommended for use following a first level of exposure of the face mask to ultraviolet light.

2. The face mask system of claim 1, wherein the first level of exposure of the face mask to the ultraviolet light corresponds to a first number of disinfection cycles performed on the face mask with the ultraviolet light.

3. The face mask system of claim 2, wherein the first number of disinfection cycles is greater than two.

4. The face mask system of any preceding claim, wherein the face mask is no longer recommended for use when a mechanical property of the face mask could be degraded following the first level of exposure to the ultraviolet light.

5. The face mask system of claim 4, wherein the mechanical property comprises fit of the face mask.

6. The face mask system of claim 4 or 5, wherein the mechanical property comprises filtering capability of the face mask.

7. The face mask system of any preceding claim, wherein
the indicator is further configured to indicate the face mask is acceptable for use following a second level of exposure of the face mask to the ultraviolet light; and
the second level of exposure of the face mask to the ultraviolet light is less than the first level of exposure of the face mask to the ultraviolet light.

8. The face mask system of any preceding claim, wherein the ultraviolet light comprises UVC light.

9. The face mask system of any preceding claim, wherein the indicator is configured to visually indicate the face mask is no longer recommended for use following the first level of exposure of the face mask to the ultraviolet light, and/or
wherein the indicator is configured to change colors following the first level of exposure of the face mask to the ultraviolet light.

10. The face mask system of any preceding claim, wherein the indicator is attached to an exterior of the face mask, and/or
wherein the indicator is integrated with the face mask, and/or
wherein the indicator comprises photochromic material.

11. The face mask system of any preceding claim, wherein the face mask comprises an N95 face mask.

12. A face mask system, comprising:
a face mask; and
an indicator arranged with the face mask and having an appearance, the indicator configured to change the appearance when a first number of treatment cycles have been performed on the face mask using ultraviolet light, and the first number of treatment cycles is greater than one.

13. The face mask system of claim 12, wherein
the indicator is further configured to maintain the appearance when a second number of treatment cycles have been performed on the face mask using the ultraviolet light; and
the second number of treatment cycles is less than the first number of treatment cycles.

14. The face mask system of claim 12 or 13, wherein the change in appearance corresponds with an increased likelihood that a mechanical property of the face mask has been degraded from exposure to the ultraviolet light.

15. A face mask system, comprising:
a face mask; and
an indicator arranged with the face mask, the indicator configured to visually indicate a mechanical property of the face mask is preserved following a first level of exposure of the face mask to ultraviolet light;
and optionally wherein:
the indicator is further configured to indicate the mechanical property of the face mask could be degraded following a second level of exposure of the face mask to the ultraviolet light; and
the second level of exposure of the face mask to the ultraviolet light is greater than the first level of exposure of the face mask to the ultraviolet light.
